**Europäisches Patentamt**

**European Patent Office**

**Office européen des brevets**

(11) Veröffentlichungsnummer: **0 118 014**
**A2**

## (12) EUROPÄISCHE PATENTANMELDUNG

(21) Anmeldenummer: **84100925.1**

(22) Anmeldetag: **30.01.84**

(51) Int. Cl.³: **G 01 N 33/00**
**G 01 N 33/20**

(30) Priorität: **08.02.83 DE 3304244**

(43) Veröffentlichungstag der Anmeldung:
**12.09.84 Patentblatt 84/37**

(84) Benannte Vertragsstaaten:
**BE DE FR GB IT NL**

(71) Anmelder: SIEMENS AKTIENGESELLSCHAFT
Berlin und München Wittelsbacherplatz 2
D-8000 München 2(DE)

(72) Erfinder: Bergfeld, Wilfried, Ing. grad.
Otto-Hahn-Strasse 78
D-4018 Langenfeld(DE)

(72) Erfinder: Ehmke, Jürgen
Mozartstrasse 23
D-5900 Siegen 31(DE)

(72) Erfinder: Körtvélyessy, László, Dipl.-Phys.
Nassauer Mauer 4
D-4190 Kleve(DE)

(72) Erfinder: Willasch, Dieter, Dr. Phys.
Marienburger Strasse 1
D-7537 Remchingen(DE)

(54) Einrichtung zur Bestimmung des Kohlenstoffgehalts in Prozessgasgemischen metallurgischer Prozesse, insbesondere in Ofenatmosphären von Wärmebehandlungsöfen.

(57) Zur Konditionierung einer Atmosphäre auf einen Gleichgewichtszustand ihres C-Gehalts wird parallel ihr Sauerstoff- und ihr Kohlendioxidgehalt gemessen und aus den Meßwerten der C-Gehalt $C_1$ ($O_2$) und $C_2$ ($CO_2$) berechnet. $C_1$ ($O_2$) folgt dem effektiven C-Gehalt $C_{eff}$ mit sehr kleiner, $C_2$ ($CO_2$) mit größerer Zeitkonstante. Die asymptotische Annäherung an den Gleichgewichtszustand (GZ) wird mit Hilfe einer Differenzbildung $\Delta C = C_1 - C_2$, die genen Null geht, überwacht, so daß dann weitere Schritte im Prozeßablauf eingeleitet werden können.

Die Meßeinrichtung wird bei metallurgischen Wärmebehand-lungsöfen, insbesondere Aufkohlungsöfen, eingesetzt, zur Überwachung der Konditionierung der Ofenatmosphäre und zur Regelung des Eintrags eines C-haltigen Reaktionspartners mit $C_1$ ($O_2$) als Istwert.

EP 0 118 014 A2

./...

FIG 1

0118014

SIEMENS AKTIENGESELLSCHAFT          Unser Zeichen
Berlin und München                  VPA 83 P 4403 E

Einrichtung zur Bestimmung des Kohlenstoffgehalts in Prozeßgasgemischen metallurgischer Prozesse, insbesondere in
Ofenatmosphären von Wärmebehandlungsöfen

Die Erfindung bezieht sich auf eine Einrichtung zur Bestimmung des Kohlenstoffgehalts in Prozeßgasgemischen metallurgischer Prozesse, insbesondere in Ofenatmosphären
von Wärmebehandlungsöfen.

Der C-Gehalt von Prozeßgasgemischen metallurgischer Prozesse, z. B. Auf- oder Entkohlen von Stahl, ist in der
Regel nicht direkt meßbar.
Eine indirekte Bestimmung ist jedoch möglich, indem die
Anteile anderer gut meßbarer Gemischkomponenten, zwischen
denen und dem C-Gehalt ein funktioneller Zusammenhang besteht, zur Berechnung herangezogen werden.

So ist es bekannt, aus dem mit einem nichtdispersiven
Infrarot-Gasanalysator (NDIR) gemessenen $CO_2$-Gehalt des
Prozeßgasgemisches dessen C-Gehalt zu bestimmen.
Da bei dieser Messung jedoch eine Querempfindlichkeit
gegen Wasserdampf besteht, geht die Feuchte der Gasatmosphäre in die Messung ein, was bei wechselnden $H_2O$-Antei-
len zu Meßwertverfälschungen führt. Der Meßwert folgt dem
effektiven C-Gehalt mit einer Verzögerung im Minutenbereich.
Nachteilig ist auch der relativ hohe Wartungsaufwand derartiger $CO_2$-Meßeinrichtungen.

Eine weitere bekannte Methode ist die Berechnung des
C-Gehalts aus den Meßwerten einer Sauerstoffmessung, vorzugsweise mit Hilfe einer sogenannten Sauerstoffsonde auf
Zirkondioxyd-Basis.

Sp 4 Scl / 03.02.1983

0118014

Diese Methode zeichnet sich durch hohe Meßgenauigkeit bei kurzer Ansprechzeit auch bei hohen Temperaturen aus, bei denen die Standzeit der Sonde allerdings geringer ist.

Bei Chargen-Prozessen, wie sie z. B. in Wärmebehandlungsöfen durchgeführt werden, muß die Ofenatmosphäre nach dem Chargieren konditioniert werden, d. h., es muß sich ein Gleichgewichtszustand einstellen, zwischen den gleichzeitig ablaufenden oxydierenden und reduzierenden Reaktionen der Prozeßgaskomponenten miteinander und mit anderen Reaktionspartnern, vor allem der eingesetzten Charge und den beim Chargieren in den Ofen gelangten Störgasen, z. B. Wasserdampf.
Erst dann können weitere Verfahrensschritte im Prozeßablauf folgen.

Da die Dauer der Konditionierung abhängig ist von der Gaszusammensetzung, von der absoluten Luftfeuchtigkeit, von der Chargenoberfläche, vom Zustand der Wärmeisolation und anderen Faktoren, war es bisher im praktischen Betrieb nicht möglich, den Zeitraum bis zum Erreichen des Gleichgewichtszustandes anhand von Messungen, insbesondere des C-Gehalts, eindeutig zu bestimmen.

Es besteht somit die Aufgabe, eine Einrichtung der eingangs genannten Art zu schaffen, die es bei hoher Ausfallsicherheit ermöglicht, beim Konditionieren einer Prozeßgasatmosphäre das Erreichen des Gleichgewichtszustandes zu erkennen, den C-Gehalt zu überwachen und eventuell zu regeln.

Mit einer erfindungsgemäßen Einrichtung gemäß Anspruch 1 läßt sich diese Aufgabe lösen.

Die zeitliche Verzögerung der C-Bestimmung aus der $CO_2$-Messung gegenüber der den Änderungen des effektiven C-Gehalts mit vernachlässigbarer Zeitkonstante folgenden aus

**0118014**

der $O_2$-Messung wird hier ausgenutzt, um die asymptotische Annäherung an den Gleichgewichtszustand durch Beobachtung des Vergleichswerts zu verfolgen, der als Differenz gegen Null oder als Quotient gegen Eins geht.

Durch die gleichzeitige Anwendung zweier unabhängiger Meßverfahren zur Bestimmung des C-Gehalts wird eine hohe Ausfallsicherheit erreicht, da bei Störungen einer Meßeinrichtung ohne Betriebsunterbrechung auf die andere umgeschaltet werden kann.

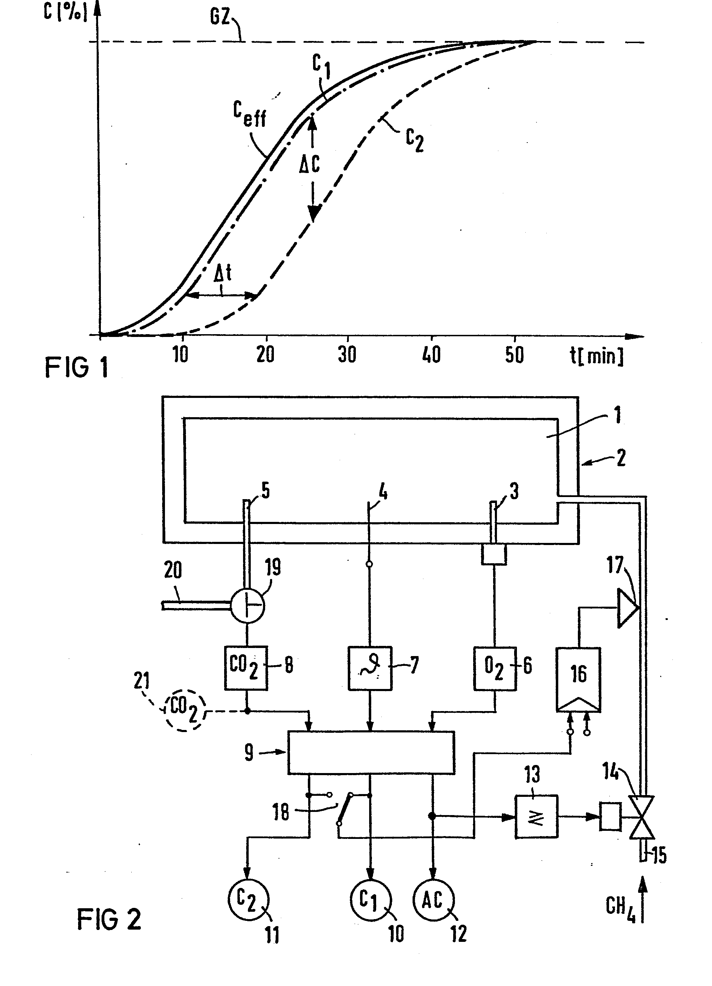

In dem Diagramm der Figur 1 sind die Zusammenhänge dargestellt, auf welchen die Wirkungsweise der erfindungsgemäßen Einrichtung beruhen.

Weitere Merkmale und Vorteile ergeben sich aus der folgenden Beschreibung eines in der Figur 2 dargestellten Ausführungsbeispiels in Verbindung mit den Unteransprüchen.

Figur 1 zeigt in einem Diagramm, in welchem der C-Gehalt in % der Ofenatmosphäre über der Zeit t aufgetragen ist, den Verlauf des effektiven und errechneten C-Gehalts der Ofenatmosphäre eines Aufkohlungsofens in der Konditionierungsphase.
Die Ofenatmosphäre enthält beispielsweise 0,1 bis 0,01 % $H_2O$, 20 % $CO$, 40 % $H_2$ und 40 % $N_2$.

Bei dem auf das Chargieren folgenden Konditionieren tritt ein Anstieg des effektiven C-Gehalts $C_{eff}$ auf, dessen Verlauf empirisch durch wiederholtes Einbringen von Metallfolien und Auswerten ihrer Kohlenstoffaufnahme festgestellt werden kann. Es ergibt sich die ausgezogene Kurve $C_{eff}$, deren Anstieg bei Annäherung an den Gleichgewichtszustand GZ flacher wird.

Die strichpunktierte Kurve $C_1$ stellt den Anstieg des C-Gehalts in der Gasatmosphäre dar, wie er sich aus den mit Hilfe einer Sauerstoffsonde gewonnenen Meßwerten als Funktion des Sauerstoff-Partialdrucks und der Temperatur errechnet.

Man erkennt, daß die Kurve $C_1 = f(O_2)$ praktisch unverzögert der Kurve $C_{eff}$ des empirisch ermittelten effektiven C-Gehalts folgt.

Die punktierte Kurve $C_2$ zeigt den Verlauf des aus dem gleichzeitig gemessenen $CO_2$-Gehalt der Ofenatmosphäre errechneten C-Gehalts. Es ist bekannt, daß in der Ungleichgewichtsphase der Ofenatmosphäre, also während des Konditionierens, die Kurve $C_2 = f(CO_2)$ mit einer zeitlichen Verzögerung $\Delta t$ im Minutenbereich der Kurve $C_{eff}$ des effektiven C-Gehalts folgt. $\Delta t$ bleibt im wesentlichen konstant.

Der Vergleich der Werte von $C_1$ und $C_2$ zu gleichen Zeitpunkten läßt erkennen, daß sie sich bei Annäherung an den Gleichgewichtszustand GZ einander ebenfalls nähern, die Differenz $\Delta C = C_1 - C_2$ wird immer kleiner.
Bei einem bestimmten Wert von $\Delta C$, beispielsweise 0,15 %, kann die Konditionierung als abgeschlossen gelten, weitere Verfahrensschritte können eingeleitet werden, z. B. die geregelte Zumischung eines kohlenstoffhaltigen Reaktionspartners.

Die Annäherung an den Gleichgewichtszustand GZ ist somit relativ bestimmbar, ohne von Prozeßvariablen, wie Chargenoberfläche, absolute Luftfeuchtigkeit, Zustand der Wärmeisolation, Türöffnungsdauer und Änderung der Gaszusammensetzung, abhängig zu sein.

Figur 2 zeigt in schematischer Darstellung eine erfindungsgemäße Einrichtung und ihre Verwendung zur Überwachung und Regelung des C-Gehalts in der Ofenatmosphäre 1 eines Aufkohlungsofens 2.

Durch die Wand des Ofens 2 ragen eine Sauerstoffsonde 3, ein Temperaturfühler 4 und eine Gasentnahme 5 in die Ofenatmosphäre 1.

Die an die Sauerstoffsonde 3 angeschlossene Meßschaltung 6 gibt ein dem Sauerstoffgehalt entsprechendes elektrisches Signal auf einen Eingang eines Rechners 9, vorzugsweise einen Mikroprozessor.

Entsprechend gibt die an den Temperaturfühler 4 angeschlossene Meßschaltung 7 ein die Temperatur $\vartheta$ abbildendes Signal und ein aus der Gasentnahme 5 gespeister NDIR-Gasanalysator ein dem $CO_2$-Gehalt der Ofenatmosphäre proportionales Signal in den Rechner 9.

Dort wird aufgrund bekannter funktioneller Zusammenhänge aus Sauerstoffgehalt und Temperatur der C-Gehalt $C_1$ sowie aus dem $CO_2$-Gehalt der C-Gehalt $C_2$ berechnet und mit benachbart angeordneten Anzeigen 10 und 11, vorzugsweise digital, angezeigt.

Das Leitstandspersonal ist so jederzeit in der Lage, Störungen in einer der Meßeinrichtungen 3, 6 bzw. 5, 8 zu erkennen.

In dem Rechner 9 wird auch fortlaufend die Differenz $C_1 - C_2$ gebildet und als Vergleichswert $\Delta C$ in einer Anzeige 12 angezeigt und/oder einem Grenzwertschalter 13 aufgeschaltet, der bei Überschreiten eines vorgegebenen Grenzwerts, der erfahrungsgemäß eine ausreichende Annäherung an den Gleichgewichtszustand signalisiert, ein Ventil 14 in der Leitung 15 aufsteuert und die Zufuhr eines kohlenstoffhaltigen Reaktionspartners, hier Methan $CH_4$, in den Ofen 2 freigibt.

Zur Regelung des Kohlenstoffeintrags ist ein Regler 16 vorgesehen, der auf ein Stellglied 17 in der Leitung 15 wirkt und dem als Regelgröße der Istwert $C_1 = f(O_2)$ zugeführt ist, der, wie bereits erwähnt, der Änderung des C-Gehalts mit vernachlässigbarer Zeitkonstante folgt.

Sollte die Sauerstoffsonde 3 ausfallen oder eine andere Störung im Zuge der Meßwertverarbeitung auftreten, so kann mit dem Schalter 18 ohne Unterbrechung des Aufkohlungsprozesses auf den Meßwert $C_2 = f(CO_2)$ umgeschaltet werden, der insbesondere nach Erreichen des Gleichgewichtszustandes einen genügend genauen Istwert für die Regelung des C-Gehalts der Ofenatmosphäre abgibt.

Wird der Meßwert $C_2$ zu bestimmten Zeiten nicht zur Bildung des Vergleichswertes $\Delta C$ benötigt, kann mittels des Dreiwegeventils 19 von der Gasentnahme 5 auf eine andere Gasentnahme 20 umgeschaltet und der NDIR-Gasanalysator 8 beispielsweise zur Bestimmung des $CO_2$-Gehalts der Abgase bei der Einstellung oder Überprüfung von Brennern für die Ofenheizung benutzt werden. Dazu ist eine weitere Anzeige 21 vorgesehen.

7 Patentansprüche
2 Figuren

Patentansprüche

1. Einrichtung zur Bestimmung des Kohlenstoffgehalts in Prozeßgasgemischen metallurgischer Prozesse, insbesondere in Ofenatmosphäre von Wärmebehandlungsöfen, g e - k e n n z e i c h n e t   d u r c h die Kombination folgender, zum Teil bekannter Merkmale:

1.    eine der Prozeßatmosphäre ausgesetzte Sauerstoff-sonde, vorzugsweise auf $ZrO_2$-Basis, zur Messung des Sauerstoffanteils;

1.1   eine Rechenschaltung zur Berechnung des C-Gehalts ($C_1$) aus dem Meßsignal der Sauerstoffsonde (3) und eines Temperaturfühlers (4);

1.2   eine Anzeige (10) des errechneten C-Gehalts ($C_1$);

2.    einen NDIR-Gasanalysator (8) zur Messung des $CO_2$-Gehalts der Prozeßatmosphäre;

2.1   eine Rechenschaltung zur Berechnung des C-Gehalts ($C_2$) der Prozeßatmosphäre aus deren $CO_2$-Gehalt;

2.2   eine Anzeige (11) des errechneten C-Gehalts ($C_2$);

3.    eine Rechenschaltung zur Bildung eines Vergleichs-werts ($\Delta C$) aus den errechneten C-Gehalten ($C_1$ und $C_2$).

2. Einrichtung nach Anspruch 1, g e k e n n z e i c h -n e t   d u r c h  die Aufschaltung einer der Ausgangsgrößen der Rechenschaltungen, vorzugsweise der Rechenschaltung nach 1.1, als Eingangsgröße "Istwert" eines den Eintrag eines Reaktionspartners in die Prozeßatmosphäre beeinflussenden Reglers (16).

3. Einrichtung nach Anspruch 2, d a d u r c h   g e -k e n n z e i c h n e t ,  daß der Reaktionspartner eine kohlenstoffhaltige Verbindung ist.

4. Einrichtung nach Anspruch 1 oder 2, g e k e n n -z e i c h n e t   d u r c h  einen Schalter (18), der im Störungsfall von der Sauerstoffmessung (3, 6) auf die $CO_2$-Messung (5, 8) umschaltet.

5. Einrichtung nach Anspruch 1, g e k e n n z e i c h - n e t   d u r c h   eine Anzeige (12) des Vergleichswerts ($\Delta C$).

6. Einrichtung nach Anspruch 1, d a d u r c h   g e - k e n n z e i c h n e t ,   daß die Gasentnahme des NDIR-Gasanalysators (8) von einem ein Prozeßgasgemisch enthaltenden Raum (1) auf einen ein zweites Prozeßgasgemisch enthaltenden Raum, insbesondere eine Abgasleitung, umschaltbar ist.

7. Einrichtung nach Anspruch 6, g e k e n n z e i c h - n e t   d u r c h   eine Anzeige (21) des $CO_2$-Gehalts.

FIG 1

FIG 2